# EUROPÄISCHE PATENTSCHRIFT

(11) **EP 2 440 518 B1**
(45) Veröffentlichungstag und Bekanntmachung des Hinweises auf die Patenterteilung: **20.08.2014**
(21) Anmeldenummer: 10722625.0
(22) Anmeldetag: 26.05.2010
(51) Int. Cl.: C07C 253/20, C07C 255/10

(54) **VERFAHREN ZUR HERSTELLUNG VON FLUORALKYLNITRILEN**
METHOD FOR MAKING FLUOROALKYL NITRILES
PROCÉDÉ DE FABRICATION DE FLUORALKYLNITRILES

(30) Priorität: 08.06.2009 EP 09162172
(43) Veröffentlichungstag der Anmeldung: 18.04.2012
(73) Patentinhaber: Bayer CropScience AG, 40789 Monheim (DE)
(72) Erfinder: LUI, Norbert, 51519 Odenthal (DE); HEINRICH, Jens-Dietmar, 51399 Burscheid (DE); WOLLNER, Thomas, 50825 Köln (DE); PAZENOK, Sergii, 42699 Solingen (DE)
(86) Internationale Anmeldenummer: PCT/EP2010/003209
(87) Internationale Veröffentlichungsnummer: WO 2010/142377

(56) Entgegenhaltungen:
- EP-A- 0 546 529
- GRUNEWALD G L ET AL: "Application of the Goldilocks effect to the design of potent and selective inhibitors of phenylethanolamine N-methyltransferase: balancing Pka and steric effects in the optimization of 3-methyl-1,2,3,4-tetrahydroisoquinoline inhibitors by beta-fluorination" JOURNAL OF MEDICINAL CHEMISTRY, USAMERICAN CHEMICAL SOCIETY. WASHINGTON, Bd. 49, Nr. 10, 1. Januar 2006 (2006-01-01), Seiten 2939-2952, XP002487200 in der Anmeldung erwähnt
- DATABASE BEILSTEIN BEILSTEIN INSTITUTE FOR ORGANIC CHEMISTRY, FRANKFURT-MAIN, DE; 2004, XP002553592 gefunden im XFIRE Database accession no. 235868 (reaction id) & SYNTHETIC COMMUNICATIONS, Bd. 34, Nr. 5, 2004, Seiten 903-908, in der Anmeldung erwähnt

## Beschreibung

Die vorliegende Erfindung betrifft ein Verfahren zur Herstellung von Fluoralkylnitrilen ausgehend von Fluoralkylcarbonsäureamiden.

Fluoralkylnitrile sind wichtige Zwischenprodukte zur Herstellung von agrochemischen Wirkstoffen.

Aus der US 2 939878 ist bekannt, dass man Fluornitrile ausgehend von Chlordifluormethan und Chlorcyan bei Temperaturen von 500 - 750°C erhalten kann. Die unselektive Reaktion führt zu Mischungen von Trifluoracetonitril, Chlordifluoracetonitril, 2-Chlortetrafluorpropionitril und weiteren fluorierten Leichtsiedern.

Eine weitere Patentanmeldung (JP 59118751 A) beschreibt die Umsetzung von Chlorfluoralkanen der Formel RFCCl₃ mit Ammoniak bei Temperaturen von 800 °C. Der gleiche Prozess wird von Hellberg und Massonne (Chemiker-Ztg./Chem. Apparatur/Verfahrenstechnik, 93. Jahrgang (1969) Nr. 6, 209 - 211) beschrieben. Bei 500 - 800 °C wird R 113a mit Ammoniak umgesetzt. Auch hierbei erhält man eine Mischung von Produkten (CF₃CN, CF₃Cl, CF₃H, CF₃CCl₃, C₂F₄Cl₂, C₂F₂Cl₄, C₂F₃Cl, CF₂Cl₂).

Grunewald et al. (J. Med. Chem. 2006, 49, 2939-2952) und auch Swarts (Bulletin Societes Chemiques Belges, 1922, Vol 31, 364- 365) beschreiben die Herstellung von Difluoracetonitril ausgehend von Difluoracetamid mit Phosphorpentoxid. Hierbei werden die beiden Feststoffe erhitzt und das leichtflüchtige Nitril bei -78°C kondensiert. Der im Reaktionsgefäß zurückbleibende feste Reaktionsrückstand ist jedoch schwer zu entfernen.

Ein weiteres Verfahren beschreibt die elektrochemische Fluorierung von Acetonitrilen (Masatake Haruta und Nobuatsu Watanabe, J. Fluorine Chemistry, 7 (1976)159-177). Die Reaktion verläuft unselektiv und man erhält auch hier Mischungen von Reaktionsprodukten.

Foulletier (EP 55651 B1) beschreibt die Gasphasenfluorierung von Trichloracetonitril bei 400 °C. Auch hierbei erhält man Mischungen.

Parker beschreibt in Synthetic Communications (Volume 34, 2004, Pages 903 - 907) die Herstellung von Trifluoracetonitril ausgehend von Trifluoracetamid mit Trifluoressigsäureanhydrid in Pyridin. Nachteilig bei diesem Prozess ist der Einsatz von teurem Trifluoressigsäureanhydrid, das stöchiometrisch eingesetzt werden muß.

Alle oben beschriebenen Verfahren sind gekennzeichnet dadurch, dass man Spezialapparaturen, sehr hohe Temperaturen, teure und gefährliche Reagenzien benötigt und die gewünschten Produkte nur durch aufwendige Isolierung aus den Produktgemischen isolieren kann.

Ausgehend von diesem Stand der Technik ergibt sich als Aufgabe der vorliegenden Erfindung, ein Verfahren zur Herstellung von fluorierten Alkylnitrilen bereitzustellen, welches vorzugsweise einfach und kostengünstig durchzuführen ist. Die mit diesem angestrebten Verfahren erhältlichen fluorierten Alkylnitrile sollen dabei vorzugsweise mit hoher Ausbeute und hoher Reinheit erhalten werden. Insbesondere soll das angestrebte Verfahren den Erhalt der gewünschten Zielverbindungen ohne die Notwendigkeit komplexer Aufreinigungsmethoden ermöglichen.

Die Aufgabe wurde gemäß der vorliegenden Erfindung gelöst durch ein Verfahren zum Herstellen von Fluoralkylnitrilen der allgemeinen Formel (I) 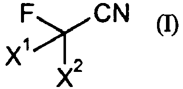 in welcher
X¹ und X² unabhängig voneinander für Fluor, Chlor, Brom, Wasserstoff, C₁₋₁₂-Alkyl, C₁₋₁₂-Halogenalkyl, C₅₋₁₈-Aryl, C₇₋₁₉-Alkylaryl oder C₇₋₁₉-Arylalkyl stehen,
dadurch gekennzeichnet, dass man
fluorierte Carbonsäureamide der Formel (II) 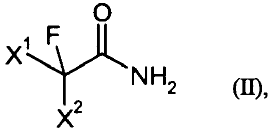
in welcher X¹ und X² die oben genannte Bedeutungen haben,
in Gegenwart einer Base und katalytischen Mengen einer fluorierten Carbonsäure der Formel (III), 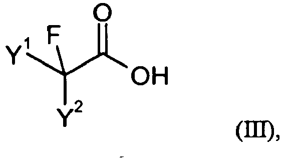
wobei
Y¹ und Y² unabhängig voneinander für Fluor, Chlor, Brom, Wasserstoff, C₁₋₁₂-Alkyl, C₁₋₁₂-Halogenalkyl, C₅₋₁₈-Aryl, C₇₋₁₉-Alkylaryl oder C₇₋₁₉-Arylalkyl stehen,
mit einem Säurehalogenid der Formel (IV) 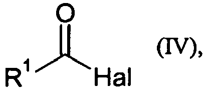
in welcher R¹ für C₁₋₁₂-Alkyl, C₃₋₈-Cycloalkyl, C₁₋₁₂-Halogenalkyl, C₅₋₁₈-Aryl, C₇₋₁₉-Arylalkyl oder C₇₋₁₉-Alkylaryl steht, und Hal für Halogen steht, umsetzt.

Bevorzugte, besonders bevorzugte und ganz besonders bevorzugte Bedeutungen der in den oben erwähnten allgemeinen Formel (I) aufgeführten Reste X¹ und X² werden im Folgenden erläutert.
X¹ und X² stehen unabhängig voneinander bevorzugt für Fluor, Chlor, Wasserstoff, C₁₋₁₂-Alkyl, C₁₁₂-Halogenalkyl oder C₅₋₁₈-Aryl,
X¹ und X² stehen unabhängig voneinander besonders bevorzugt für Fluor, Chlor, Wasserstoff oder C₁₋₁₂-Halogenalkyl,
X¹ und X² stehen unabhängig voneinander ganz besonders bevorzugt für Fluor, Wasserstoff oder C₁₋₁₂-Halogenalkyl.

Überraschenderweise lassen sich die fluorierten Alkylnitrile der Formel (I) unter den erfindungsgemäßen Bedingungen mit guten Ausbeuten in hoher Reinheit herstellen, womit das erfindungsgemäße Verfahren die im Zusammenhang mit dem Stand der Technik beschriebenen Nachteile nicht aufweist.

Das erfindungsgemäße Verfahren kann anhand des folgenden Schema (I) erläutert werden : 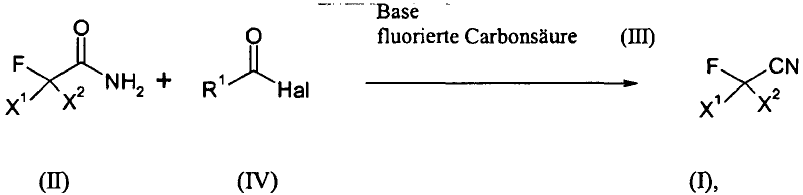 wobei X¹, X², R¹, Hal die oben angegebenen Bedeutungen haben.

### Schema (I)

### Allgemeine Definitionen

Im Zusammenhang mit der vorliegenden Erfindung umfasst der Begriff Halogene (Hal), soweit nicht anders definiert, solche Elemente, die ausgewählt sind aus der Gruppe bestehend aus Fluor, Chlor, Brom und Iod, wobei Fluor, Chlor und Brom bevorzugt und Fluor und Chlor besonders bevorzugt verwendet werden.

Gegebenenfalls substituierte Gruppen können einfach oder mehrfach substituiert sein, wobei bei Mehrfachsubstitutionen die Substituenten gleich oder verschieden sein können.

Mit einem oder mehreren Halogenatomen (-Hal) substituierte Alkyl-Gruppen sind beispielsweise ausgewählt aus Trifluormethyl (CF₃), Difluormethyl (CHF₂), CF₃CH₂, ClCH₂, CF₃CCl₂.

Alkyl-Gruppen sind im Zusammenhang mit der vorliegenden Erfindung, soweit nicht abweichend definiert, lineare, verzweigte oder ringförmige gesättigte Kohlenwasserstoff-Gruppen.

Die Definition C₁-C₁₂-Alkyl umfasst den größten hierin definierten Bereich für eine Alkyl-Gruppe. Im Einzelnen umfasst diese Definition beispielsweise die Bedeutungen Methyl, Ethyl, n-, isoPropyl, n-, iso-, sec- und t-Butyl, n-Pentyl, n-Hexyl, 1,3-Dimethylbutyl, 3,3-Dimethylbutyl, n-Heptyl, n-Nonyl, n-Decyl, n-Undecyl, n-Dodecyl.

Aryl-Gruppen sind im Zusammenhang mit der vorliegenden Erfindung, soweit nicht abweichend definiert, aromatische Kohlenwasserstoff-Gruppen, die ein, zwei oder mehrere Heteroatome, die ausgewählt sind aus O, N, P und S, aufweisen können.

Die Definition C₅₋₁₈Aryl umfasst den größten hierin definierten Bereich für eine Aryl-Gruppe mit 5 bis 18 Gerüst-Atomen, wobei die C-Atome gegen Heteroatome ausgetauscht sein können. Im Einzelnen umfasst diese Definition beispielsweise die Bedeutungen Cyclopentadienyl, Phenyl, Cycloheptatrienyl, Cyclooctatetraenyl, Naphthyl und Anthracenyl; 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl; 1-Pyrrolyl, 1-Pyrazolyl, 1,2,4-Triazol-1-yl, 1-Imidazolyl, 1,2,3-Triazol-1-yl, 1,3,4-Triazol-1-yl; 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl. Arylalkyl-Gruppen (Aralkyl-Gruppen) sind im Zusammenhang mit der vorliegenden Erfindung, soweit nicht abweichend definiert, durch Aryl-Gruppen substituierte Alkyl-Gruppen, die eine C₁₋₈-Alkylenkette aufweisen können und im Arylgerüst ein oder mehrere Heteroatome, die ausgewählt sind aus O, N, P und S, aufweisen können.

Die Definition C₇₋₁₉-Aralkyl-Gruppe umfasst den größten hierin definierten Bereich für eine Arylalkyl-Gruppe mit insgesamt 7 bis 19 Atomen in Gerüst und Alkylenkette. Im Einzelnen umfasst diese Definition beispielsweise die Bedeutungen Benzyl und Phenylethyl.

Alkylaryl-Gruppen (Alkaryl-Gruppen) sind im Zusammenhang mit der vorliegenden Erfindung, soweit nicht abweichend definiert, durch Alkyl-Gruppen substituierte Aryl-Gruppen, die eine C₁₋₈-Alkylenkette aufweisen können und im Arylgerüst ein oder mehrere Heteroatome, die ausgewählt sind aus O, N, P und S, aufweisen können.

Die Definition C₇₋₁₉-Alkylaryl-Gruppe umfasst den größten hierin definierten Bereich für eine Alkylaryl-Gruppe mit insgesamt 7 bis 19 Atomen in Gerüst und Alkylenkette. Im Einzelnen umfasst diese Definition beispielsweise die Bedeutungen Tolyl-, 2,3-, 2,4-, 2,5-, 2,6-, 3,4- oder 3,5-Dimethylphenyl.

### Fluorierte Carbonsäuren (III)

Die gemäß der vorliegenden Erfindung verwendeten fluorierten Carbonsäuren sind Verbindungen der allgemeinen Formel (III) 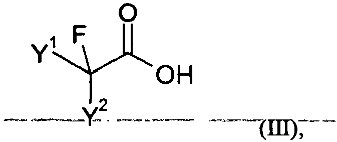 in welcher
Y¹ und Y² unabhängig voneinander für Fluor, Chlor, Brom, Wasserstoff, C₁₋₁₂-Alkyl, C₁₋₁₂-Halogenalkyl, C₅₋₁₈-Aryl, C₇₋₁₉-Alkylaryl oder C₇₋₁₉-Arylalkyl, bevorzugt für Wasserstoff, Fluor, Chlor, C₂₋₈-Alkyl oder C₂₋₈-Halogenalkyl, besonders bevorzugt für Fluor, Chlor, Wasserstoff, C₃₋₆-Alkyl, CF₃ oder CF₂H stehen.

Beispiele für erfindungsgemäß geeignete fluorierte Carbonsäuren sind Trifluoressigsäure, Difluoressigsäure, Difluorchloressigsäure, Chlorfluoressigsäure, 2,3,3,3-Tetrafluorpropionsäure, 2,2,3,3-Tetrafluorpropionsäure, 2,2-Difluorpropionsäure, Pentafluorpropionsäure, 2,3,3,4,4,4-Hexafluorbutancarbonsäure.

Das molare Verhältnis von fluorierter Carbonsäure der Formel (III) zum eingesetzten fluorierten Alkylamid der Formel (II) kann beispielsweise 0,05 bis 1 betragen, bevorzugt 0,11 bis 0,8, besonders bevorzugt 0,2 bis 0,7. Der Einsatz größerer Mengen (größere molare Verhältnisse als 1) an fluorierter Carbonsäure ist unkritisch aber unwirtschaftlich.

### Säurehalogenide (IV)

Die zuvor beschriebenen fluorierten Alkylamide der Formel (II) werden unter Zugabe eines Säurehalogenids der Formel (IV) umgesetzt.

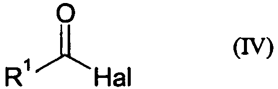

In Formel (IV) ist R¹ ausgewählt aus C₁₋₁₂-Alkyl, C₃₋₈-Cycloalkyl, C₁₋₁₂-Halogenalkyl, C₅₋₁₈-Aryl, C₇₋₁₉-Arylalkyl oder C₇₋₁₉-Alkylaryl, bevorzugt aus C₅₋₁₈-Aryl oder C₂₋₈-Alkyl, besonders bevorzugt aus C₃₋₆-Alkyl oder C₆-Aryl,
und Hal steht für Fluor, Chlor, Brom oder Iod, bevorzugt für Chlor oder Brom, besonders bevorzugt für Chlor.

Beispiele für erfindungsgemäß geeignete Säurehalogenide sind Essigsäurechlorid, Pivalinsäurechlorid, 2,2-Dimethylbuttersäurechlorid, Isovaleroylchlorid und Benzoylchlorid.

Das molare Verhältnis von Säurehalogeniden der Formel (IV) zum eingesetzten fluorierten Alkylamid der Formel (II) kann beispielsweise 0,5 bis 5 betragen, bevorzugt 1 bis 3, besonders bevorzugt 2 bis 2,5.

### Basen

Das erfindungsgemäße Verfahren wird in Gegenwart einer Base durchgeführt. Als Basen eignen sich beispielsweise substituierte oder unsubstituierte Pyridine und substituierte oder unsubstituierte Chinoline. Vorzugsweise werden substituierte oder unsubstituierte Pyridine und substituierte oder unsubstituierte Chinoline eingesetzt.

Bevorzugte Beispiele für Basen sind Pyridin, 3-, 4-Picolin, Chinolin, Chinaldin, halogenierte Pyridine. Besonders bevorzugt werden Pyridin, 4-Picolin, 2-Chlorpyridin verwendet.

Das molare Verhältnis von Base zur eingesetzten fluorierten Carbonsäure der Formel (III) kann beispielsweise 0,5 bis 10 betragen, bevorzugt 1 bis 8, besonders bevorzugt 1,5 bis 6.

Der Einsatz größerer Mengen an Base ist unkritisch aber unwirtschaftlich.

Die Umsetzung zur Herstellung der Verbindungen der allgemeinen Formel (I) kann im Allgemeinen im Vakuum, bei Normaldruck oder unter Überdruck durchgeführt werden. Die angewendeten Temperaturen können ebenfalls, in Abhängigkeit der verwendeten Substrate, variieren und sind für den Fachmann durch Routineversuche leicht zu ermitteln. Beispielsweise kann die Umsetzung zur Herstellung der Verbindungen der allgemeinen Formel (I) bei einer Temperatur von -50 bis 250 °C, vorzugsweise 0 bis 170 °C, durchgeführt werden. Besonders bevorzugt wird die Umsetzung bei Temperaturen von 10 bis 140 °C durchgeführt.

Die gemäß der vorliegenden Erfindung verwendeten fluorierten Alkylamide der Formel (II) sind kommerziell erhältlich oder können nach literaturbekannten Verfahren leicht hergestellt werden (WO 03/080563).

### Lösungsmittel

Die Umsetzung des fluorierten Alkylamides der Formel (II) zur Verbindung mit der Formel (I) kann in Gegenwart eines Lösungsmittels durchgeführt werden. Vorzugsweise wird bei der Reaktion auf ein zusätzliches Lösungsmittel verzichtet. Als geeignete Lösungsmittel sind zu nennen: Halogenkohlenwasserstoffe und aromatische Kohlenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol; Ether, wie Ethylpropylether, *n*-Butylether, Anisol, Phenetol, Cyclohexylmethylether, Dimethylether, Diethylether, Dimethylglycol, Diphenylether, Dipropylether, Diisopropylether, Di-*n*-butylether, Diisobutylether, Diisoamylether, Ethylenglycoldimethylether, Isopropylethylether, Methyl-tert-butylether, Tetrahydrofuran, Methyl-Tetrahydrofuran, Dioxan, Dichlordiethylether und Polyether des Ethylenoxids und/oder Propylenoxids; Nitrokohlenwasserstoffe wie Nitromethan, Nitroethan, Nitropropan, Nitrobenzol, Chlornitrobenzol, o-Nitrotoluol; aliphatische, cycloaliphatische oder aromatische Kohlenwasserstoffe wie Pentan, n-Hexan, n-Heptan, n-Oktan, Nonan beispielsweise sogenannte White Spirits mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Petrolether, Ligroin, Octan, Benzol, Toluol, Xylol; Ester wie Methyl-, Ethyl-, Butyl-, Isobutylacetat, sowie Dimethyl-, Dibutyl-, Ethylencarbonat . Bevorzugte Lösungsmittel sind Toluol oder Chlorbenzole.

Die Isolierung der gewünschten Verbindungen der allgemeinen Formel (I) kann beispielsweise durch Destillation erfolgen. Hierbei kann das Produkt (I) während der Zudosierung des Säurechlorides der Formel (IV) gleichzeitig abdestilliert werden. Eine weitere Isoliervariante ist erst nach Reaktionsende das Produkt der Formel (I) durch Destillation oder Filtration abzutrennen.

Die vorliegende Erfindung wird anhand der nachfolgenden Beispiele näher erläutert.

### Herstellungsbeispiele

### Beispiel 1

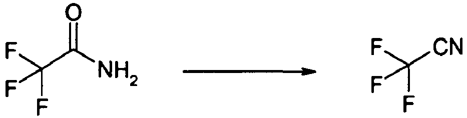

4,6 g Trifluoressigsäure werden in 48,9 g Pyridin vorgelegt und mit 13,4 g Trifluoressigsäureamid versetzt. Zu dieser Lösung wird dann über 5 Stunden 30,4 g 2,2-Dimethylpropanoylchlorid bei Raumtemperatur zugetropft. Es entsteht beim Zutropfen sofort das gasförmige Trifluoracetonitril. Dieses kann bei tieferen Temperaturen (z.B. bei -100°C) kondensiert werden oder direkt in eine Folgereaktion eingeleitet werden. Die Ausbeute beträgt 92 %.

### Beispiel 2

2 g Difluoressigsäure werden in 34,8 g Pyridin vorgelegt und mit 5 g Trifluoressigsäureamid versetzt. Zu dieser Lösung wird dann über 2 Stunden 10,9 g 2,2-Dimethylpropanoylchlorid bei Raumtemperatur zugetropft. Es entsteht beim Zutropfen sofort das gasförmige Trifluoracetonitril. Die Ausbeute beträgt 90 %.

### Beispiel 3

2,4 g Trifluoressigsäure werden in 34,8 g Pyridin vorgelegt und mit 5 g Trifluoressigsäureamid versetzt. Zu dieser Lösung wird dann über 2 Stunden 12,4 g 2,2-Dimethylbuttersäurechlorid bei Raumtemperatur zugetropft. Es entsteht beim Zutropfen sofort das gasförmige Trifluoracetonitril. Die Ausbeute beträgt 84 %.

### Beispiel 4

4,6 g Trifluoressigsäure werden in 48,9 g Pyridin vorgelegt und mit 13,4 g Trifluoressigsäureamid versetzt. Zu dieser Lösung wird dann über 5 Stunden 35,5 g Benzoylchlorid bei Raumtemperatur zugetropft. Es entsteht beim Zutropfen sofort das gasförmige Trifluoracetonitril. Die Ausbeute beträgt 86 %.

### Beispiel 5

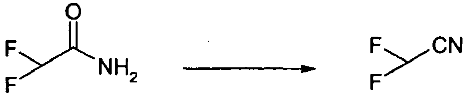

11,6 g Trifluoressigsäure werden in 146,7 g Pyridin vorgelegt und mit 19,2 g Difluoressigsäureamid versetzt. Zu dieser Lösung wird dann über 3 Stunden 51,2 g 2,2-Dimethylpropanoylchlorid bei 60°C zugetropft. Anschließend wird die Reaktionsmischung auf 100°C erhitzt und das Difluoracetonitril abdestilliert. Man erhält das Difluoracetonitril in einer Ausbeute von 88 %.

### Beispiel 6

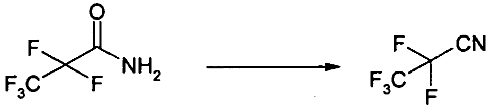

5,5 g Trifluoressigsäure werden in 78 g Pyridin vorgelegt und mit 16,1 g Pentafluorpropionsäureamid versetzt. Zu dieser Lösung wird dann über 4 Stunden 24,4 g 2,2-Dimethylpropanoylchlorid bei 30°C zugetropft. Es entsteht beim Zutropfen sofort das gasförmige Pentafluorpropionitril. Dieses kann bei tieferen Temperaturen (z.B. bei -80°C) kondensiert werden oder direkt in eine Folgereaktion eingeleitet werden. Die Ausbeute beträgt 82 %.

## Patentansprüche

1. Verfahren zum Herstellen von Fluoralkylnitrilen der allgemeinen Formel (I), 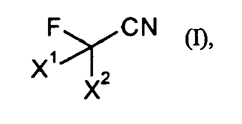 in welcher
X¹ und X² unabhängig voneinander für Fluor, Chlor, Brom, Wasserstoff, C₁₋₁₂-Alkyl, C₁₋₁₂-Halogenalkyl, C₅₋₁₈-Aryl, C₇₋₁₉-Alkylaryl oder C₇₋₁₉-Arylalkyl stehen,
**dadurch gekennzeichnet, dass** man
fluorierte Carbonsäureamide der Formel (II) 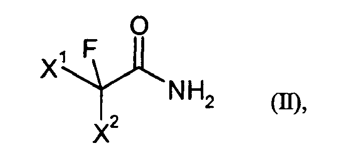
in welcher X¹ und X² die oben genannten Bedeutungen haben,
in Gegenwart einer Base und katalytischen Mengen einer fluorierten Carbonsäure der Formel (III), 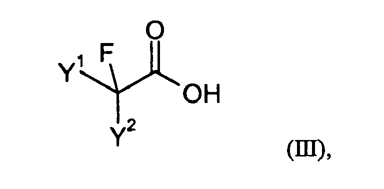
wobei
Y¹ und Y² unabhängig voneinander für Fluor, Chlor, Brom, Wasserstoff, C₁₋₁₂-Alkyl, C₁₋₁₂-Halogenalkyl, C₅₋₁₈-Aryl, C₇₋₁₉-Alkylaryl oder C₇₋₁₉-Arylalkyl stehen,
mit einem Säurehalogenid der Formel (IV) 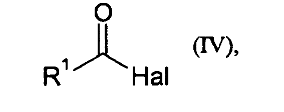
in welcher R¹ für C₁₋₁₂-Alkyl, C₃₋₈-Cycloalkyl, C₁₋₁₂-Halogenalkyl, C₅₋₁₈-Aryl, C₇₋₁₉-Arylalkyl oder C₇₋₁₉-Alkylaryl steht, und Hal für Halogen steht,
umsetzt.

2. Verfahren gemäß Anspruch 1, wobei
X¹ und X² unabhängig voneinander für Fluor, Chlor, Wasserstoff, C₁₋₁₂-Alkyl, C₁₋₁₂-Halogenalkyl oder C₅₋₁₈-Aryl stehen,
Y¹ und Y² unabhängig voneinander für Wasserstoff, Fluor, Chlor, C₂₋₈-Alkyl oder C₂₋₈-Halogenalkyl stehen,
Hal für Chlor oder Brom steht;
R¹ für C₂₋₈-Alkyl oder C₅₋₁₈-Aryl steht.

3. Verfahren gemäß Anspruch 1 oder 2, wobei
X¹ und X² unabhängig voneinander für Fluor, Chlor, Wasserstoff oder C₁₋₁₂-Halogenalkyl stehen,
Y¹ und Y² unabhängig voneinander Fluor, Chlor, Wasserstoff, C₃₋₆-Alkyl, CF₃ oder CF₂H stehen,
R¹ für C₃₋₆-Alkyl oder C₆-Aryl steht,
und Hal für Chlor steht.

4. Verfahren gemäß einem der Ansprüche 1 bis 3, wobei die fluorierten Carbonsäuren ausgewählt sind aus der Gruppe bestehend aus Trifluoressigsäure, Difluoressigsäure, Difluorchloressigsäure, Chlorfluoressigsäure, 2,3,3,3-Tetrafluorpropionsäure, 2,2,3,3-Tetrafluorpropionsäure, 2,2-Difluorpropionsäure, Pentafluorpropionsäure, 2,3,3,4,4,4-Hexafluorbutancarbonsäure.

5. Verfahren gemäß einem der Ansprüche 1 bis 4, wobei die Säurehalogenide ausgewählt sind aus der Gruppe bestehend aus Essigsäurechlorid, Pivalinsäurechlorid, 2,2-Dimethylbuttersäurechlorid, Isovaleroylchlorid, Benzoylchlorid.

6. Verfahren gemäß einem der Ansprüche 1 bis 5, wobei das molare Verhältnis von fluorierten Carbonsäuren der allgemeinen Formel (III) zum eingesetzten fluorierten Alkylamid der allgemeinen Formel (II) 0,05 bis 1 ist.

7. Verfahren gemäß einem der Ansprüche 1 bis 6, wobei das molare Verhältnis von Säurehalogeniden der allgemeinen Formel (IV) zum eingesetzten fluorierten Alkylamid der allgemeinen Formel (II) 0,5 bis 5 ist.

8. Verfahren gemäß einem der Ansprüche 1 bis 7, wobei die Base ausgewählt ist aus der Gruppe bestehend aus Pyridin, 3-, 4-Picolin, Chinolin, Chinaldin, halogenierte Pyridine.

9. Verfahren gemäß einem der Ansprüche 1 bis 8, wobei das molare Verhältnis von Base zur eingesetzten fluorierten Carbonsäure der allgemeinen Formel (III) 0,5 bis 10 ist.

## Claims

1. Process for preparing fluoroalkyl nitriles of the general formula (I), 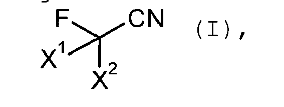 in which
X¹ and X² are each independently fluorine, chlorine, bromine, hydrogen, C₁₋₁₂-alkyl, C₁₋₁₂-haloalkyl, C₅₋₁₈-aryl, C₇₋₁₉-alkylaryl or C₇₋₁₉-arylalkyl,
**characterized in that**
fluorinated carboxamides of the formula (II) 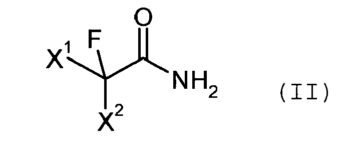
in which X¹ and X² are each as defined above,
in the presence of a base and of catalytic amounts of a fluorinated carboxylic acid of the formula (III) 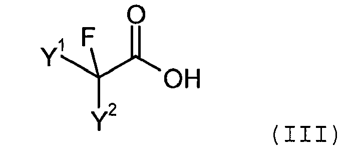 where
Y¹ and Y² are each independently fluorine, chlorine, bromine, hydrogen, C₁₋₁₂-alkyl, C₁₋₁₂-haloalkyl, C₅₋₁₈-aryl, C₇₋₁₉-alkylaryl or C₇₋₁₉-arylalkyl,
are reacted with an acid halide of the formula (IV) 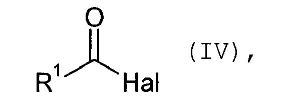 in which R¹ is C₁₋₁₂-alkyl, C₃₋₈-cycloalkyl, C₁₋₁₂-haloalkyl, C₅₋₁₈-aryl, C₇₋₁₉-arylalkyl or C₇₋₁₉-alkylaryl, and Hal is halogen.

2. Process according to Claim 1, wherein
X¹ and X² are each independently fluorine, chlorine, hydrogen, C₁₋₁₂-alkyl, C₁₋₁₂-haloalkyl or C₅₋₁₈-aryl,
Y¹ and Y² are each independently hydrogen, fluorine, chlorine, C₂₋₈-alkyl or C₂₋₈-haloalkyl,
Hal is chlorine or bromine;
R¹ is C₂₋₈-alkyl or C₅₋₁₈-aryl.

3. Process according to Claim 1 or 2, wherein
X¹ and X² are each independently fluorine, chlorine, hydrogen or C₁₋₁₂-haloalkyl,
Y¹ and Y² are each independently fluorine, chlorine, hydrogen, C₃₋₆-alkyl, CF₃ or CF₂H,
R¹ is C₃₋₆-alkyl or C₆-aryl,
and Hal is chlorine.

4. Process according to any of Claims 1 to 3, wherein the fluorinated carboxylic acids are selected from the group consisting of trifluoroacetic acid, difluoroacetic acid, difluorochloroacetic acid, chlorofluoroacetic acid, 2,3,3,3-tetrafluoropropionic acid, 2,2,3,3-tetrafluoropropionic acid, 2,2-difluoropropionic acid, pentafluoropropionic acid, 2,3,3,4,4,4-hexafluorobutanecarboxylic acid.

5. Process according to any of Claims 1 to 4, wherein the acid halides are selected from the group consisting of acetyl chloride, pivaloyl chloride, 2,2-dimethylbutyryl chloride, isovaleryl chloride, benzoyl chloride.

6. Process according to any of Claims 1 to 5, where the molar ratio of fluorinated carboxylic acids of the general formula (III) to the fluorinated alkyl amide of the general formula (II) used is 0.05 to 1.

7. Process according to any of Claims 1 to 6, wherein the molar ratio of acid halides of the general formula (IV) to the fluorinated alkyl amide of the general formula (II) used is 0.5 to 5.

8. Process according to any of Claims 1 to 7, wherein the base is selected from the group consisting of pyridine, 3-, 4-picoline, quinoline, quinaldine, halogenated pyridines.

9. Process according to any of Claims 1 to 8, wherein the molar ratio of base to the fluorinated carboxylic acid of the general formula (III) used is 0.5 to 10.

## Revendications

1. Procédé de fabrication de fluoroalkylnitriles de formule générale (I) 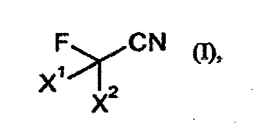 dans laquelle
X¹ et X² représentent indépendamment l'un de l'autre fluor, chlore, brome, hydrogène, alkyle en C₁₋₁₂, halogénoalkyle en C₁₋₁₂, aryle en C₅₋₁₈, alkylaryle en C₇₁₉ ou arylalkyle en C₇₋₁₉,
**caractérisé en ce que**
des amides d'acides carboxyliques fluorés de formule (II) 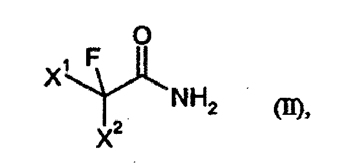
dans laquelle X¹ et X² ont les significations indiquées précédemment,
sont mis en réaction en présence d'une base et de quantités catalytiques d'un acide carboxylique fluoré de formule (III) 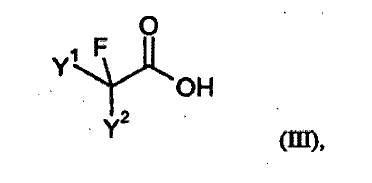 dans laquelle
Y¹ et Y² représentent indépendamment l'un de l'autre fluor, chlore, brome, hydrogène, alkyle en C₁₋₁₂, halogénoalkyle en C₁₋₁₂, aryle en C₅₋₁₈, alkylaryle en C₇₁₉ ou arylalkyle en C₇₋₁₉,
avec un halogénure d'acide de formule (IV) 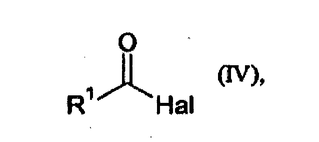
dans laquelle R¹ représente alkyle en C₁₋₁₂, cycloalkyle en C₃₋₈, halogénoalkyle en C₁₋₁₂, aryle en C₅₋₁₈, arylalkyle en C₇₋₁₉ ou alkylaryle en C₇₋₁₉, et Hal représente halogène.

2. Procédé selon la revendication 1, dans lequel
X¹ et X² représentent indépendamment l'un de l'autre fluor, chlore, hydrogène, alkyle en C₁₋₁₂, halogénoalkyle en C₁₋₁₂ ou aryle en C₅₋₁₈,
Y¹ et Y² représentent indépendamment l'un de l'autre hydrogène, fluor, chlore, alkyle en C₂₋₈ ou halogénoalkyle en C₂₋₈,
Hal représente chlore ou brome ;
R¹ représente alkyle en C₂₋₈ ou aryle en C₅₋₁₈.

3. Procédé selon la revendication 1 ou 2, dans lequel
X¹ et X² représentent indépendamment l'un de l'autre fluor, chlore, hydrogène ou halogénoalkyle en C₁₋₁₂,
Y¹ et Y² représentent indépendamment l'un de l'autre fluor, chlore, hydrogène, alkyle en C₃₋₆, CF₃ ou CF₂H,
R¹ représente alkyle en C₃₋₆ ou aryle en C₆,
et Hal représente chlore.

4. Procédé selon l'une quelconque des revendications 1 à 3, dans lequel les acides carboxyliques fluorés sont choisis dans le groupe constitué par l'acide trifluoroacétique, l'acide difluoroacétique, l'acide difluorochloroacétique, l'acide chlorofluoroacétique, l'acide 2,3,3,3-tétrafluoropropionique, l'acide 2,2,3,3-tétrafluoropropionique, l'acide 2,2-difluoropropionique, l'acide pentafluoropropionique, l'acide 2,3,3,4,4,4-hexafluorobutanecarboxylique.

5. Procédé selon l'une quelconque des revendications 1 à 4, dans lequel les halogénures d'acides sont choisis dans le groupe constitué par le chlorure d'acide acétique, le chlorure d'acide pivalique, le chlorure d'acide 2,2-diméthylbutyrique, le chlorure d'isovaléroyle, le chlorure de benzoyle.

6. Procédé selon l'une quelconque des revendications 1 à 5, dans lequel le rapport en moles entre les acides carboxyliques fluorés de formule générale (III) et l'alkylamide fluoré utilisé de formule générale (II) est de 0,05 à 1.

7. Procédé selon l'une quelconque des revendications 1 à 6, dans lequel le rapport en moles entre les halogénures d'acides de formule générale (IV) et l'alkylamide fluoré utilisé de formule générale (II) est de 0,5 à 5.

8. Procédé selon l'une quelconque des revendications 1 à 7, dans lequel la base est choisie dans le groupe constitué par la pyridine, la 3-, 4-picoline, la quinoline, la quinaldine, les pyridines halogénées.

9. Procédé selon l'une quelconque des revendications 1 à 8, dans lequel le rapport en moles entre la base et l'acide carboxylique fluoré utilisé de formule générale (III) est de 0,5 à 10.
